**Europäisches Patentamt**

**European Patent Office**

**Office européen des brevets**

(19)

(11) Veröffentlichungsnummer: **0 317 808 B1**

(12) # EUROPÄISCHE PATENTSCHRIFT

(45) Veröffentlichungstag der Patentschrift:
16.10.91 Patentblatt 91/42

(51) Int. Cl.⁵ : **A61M 5/14**

(21) Anmeldenummer: **88118369.3**

(22) Anmeldetag: **04.11.88**

(54) **Infusionsspritzenpumpe.**

(30) Priorität: **22.11.87 DE 3739563**

(43) Veröffentlichungstag der Anmeldung:
**31.05.89 Patentblatt 89/22**

(45) Bekanntmachung des Hinweises auf die
Patenterteilung:
**16.10.91 Patentblatt 91/42**

(84) Benannte Vertragsstaaten:
**DE ES FR GB IT**

(56) Entgegenhaltungen:
**EP-A- 0 176 286**
**DE-A- 2 922 037**
**GB-A- 2 094 628**
**US-A- 3 701 345**
**US-A- 4 345 483**

(73) Patentinhaber: **Fresenius AG**
**Gluckensteinweg 5**
**W-6380 Bad Homburg v.d.H. (DE)**

(72) Erfinder: **Faeser, Ulrich, Dipl.-ing.**
**Brunnenweg 2**
**W-6242 Kronberg 2 (DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**W-6200 Wiesbaden (DE)**

**Beschreibung**

Die Erfindung betrifft eine Infusionsspritzenpumpe mit einer Motor-Getriebe-Einheit zum Antrieb eines linear bewegbaren Antriebsteils mit einer Infusionsspritze, die einen Spritzenkolben aufweist, der zu seiner Betätigung mit dem Antriebsteil in Eingriff steht und mit einer Positionserfassungseinrichtung zur Bestimmung der Stellung des Spritzenkolbens, die ein positionsgebendes Element und einen ortsfesten Absolutwegaufnehmer aufweist.

Aus der DE-A 2922037, die den Obergriff des Anspruchs 1 bildes und dem DE-U 8520376 sind Spritzenpumpen bekannt, die mit optoelektronischen Erfassungseinrichtungen arbeiten. Diese Erfassungseinrichtungen weisen den Nachteil auf, daß kein stetiges Signal erzeugt werden kann, so daß die Genauigkeit der Positionserfassung eine Funktion der jeweiligen codierungsabhängigen Quantisierung der optoelektronischen Elemente ist.

Die US-PS 4,620,848 beinhaltet eine Infusionsspritzenpumpe, bei der eine stationär an einem Gehäuse angeordnete erste Rolle mit einem Spritzenkolben in Eingriff steht, der wiederum von einem Motor, einem Getriebe und einer zweiten Rolle angetrieben wird, die ebenfalls am Gehäuse gelagert ist und die mit dem Motor über das Getriebe antriebsverbunden ist. Die erste Rolle, die rotadorisch angetrieben wird, ist am Gehäuse und nicht am Antriebsteil für den Spritzenkolben gelagert.

Es ist weiterhin eine Infusionsspritzenpumpe aus der US-PS 3,701,345 bekannt, bei der ein Motor einen Kolben zum Austreiben des Fluids aus einer Spritzenhülse antreibt. Diese angiographische Infusionsspritzenpumpe weist ein Positionssteuersystem auf, das ein Rückführspannungssignal verwendet, das nicht der Spritzenkolbengeschwindigkeit, sondern der Infusionsspritzenkolbenposition zugeordnet ist. Dieses Rückführsignal wird durch ein mechanisch angetriebenes Potentiometer erzeugt, das durch den Antriebsmotor über ein zusätzliches Getriebe in Funktion gesetzt wird. Vor Durchführung der Injektion wird die momentane Position des Spritzenkolbens abgetastet und dessen Position als Basisbezugsgröße bzw. Nullgrenze herangezogen. Spannungsproportional werden vom Bediener das gewünschte Injektionsvolumen, entsprechende Spannungsgrenzen oder die gewünschte Rate vorbestimmt.

Bei diesem System wird — wie vorstehend erläutert — ein Potentiometer verwendet, um die momentane Position des Spritzenkolbens zu erfassen. Das Positionssignal wird mit einem Steuersignal verglichen und die sich daraus ergebende Differenz wird zum Betreiben des Motors verwendet. Der Motor wird durch eine Spannungsbegrenzungsschaltung beeinflußt, die verhindert, daß der Motor eine überhöhte Leistung aufbringt. Bei Rückführung des Spritzenkolbens in seine Ausgangsstellung wird die Drehrichtung des Motors, und damit des Getriebes, umgekehrt. Bei derartigen Infusionsspritzenpumpen, bei denen der Spritzenkolben in Abhängigkeit von der Motor-Getriebe-Einheit durch Umkehrung der Motordrehrichtung in seine Ausgangsstellung zurückgeführt wird, kann zwar ein momentaner Absolutpositionswert des Spritzenkolbens jederzeit ermittelt werden, jedoch ist der Aufbau des Positionssteuerungssystems dadurch kompliziert, daß eine zusätzliche Getriebeübersetzung für das Positionssteuerungssystem notwendig ist. Durch das Vorhandensein einer zusätzlichen Getriebeübersetzung kann dieses Steuersystem zudem noch fehlerbehaftet sein, da durch Ungenauigkeiten bzw. Fehler der zusätzlichen Getriebeübersetzung eine Abweichung der tatsächlichen Position des Spritzenkolbens im Spritzenzylinder von der vom Positionssteuerungssystem gelieferten Positionsinformation nicht ausgeschlossen werden kann.

Zusätzlich ist eine solche Pumpe kompliziert zu handhaben, da stets die gesamte Antriebsanordnung in den Ausgangszustand überführt werden muß, wenn eine neue (gefüllte) Spritze eingelegt werden soll.

Bei anderen bekannten Infusionsspritzenpumpen, bei denen der Spritzenkolben unabhängig von der Motor-Getriebe-Einheit in seine Nullstellung zurückgeführt werden kann, wie beispielsweise in der DE-A 3428655 beschrieben, wäre eine solche Positionswertbestimmung wünschenswert.

Aufgabe der Erfindung ist es daher, eine Infusionsspritzenpumpe gemäß Oberbegriff des Anspruch 1 zu schaffen, die permanent die momentane Absolutposition des Spritzenkolbens anzeigt.

Erfindungsgemäß wird die Aufgabe dadurch gelöst, daß das positionsgebende Element mit dem linear bewegbaren Antriebsteil starr verbunden ist und daß das positionsgebende Element entlang seiner der Verschiebungsrichtung des Antriebsteils entsprechenden Bewegungsbahn mit dem Absolutwegaufnehmer elektrisch oder magnetisch gekoppelt ist.

In einer bevorzugten Ausbildungsform ist der Absolutwegaufnehmer als Linearpotentiometer ausgebildet.

Außerdem kann der Absolutwegaufnehmer ein induktives, kapazitives oder magnetisches positionsgebendes Element sein.

Weiterhin ist der Absolutwegaufnehmer mit einer Auswerteeinheit verbunden.

Infolge der vorzugsweise starren Verbindung des positionsgebenden Elementes mit dem den Spritzenkolben antreibenden Antriebsteil kann die in der US-PS 3,701,345 mögliche Fehlerquelle des defekten Zusatzgetriebes ausgeschlossen werden. Demzufolge ist wegen des direkten Abgriffs der momentanen Kolbenposition eine weitestgehende Fehlerfreiheit gewährleistet.

Infusionsspritzenpumpen, die unabhängig von der Motor-Getriebe-Einheit den Spritzenkolben in eine beliebige Ausgangsstellung bewegen können, können mit einer in der Motor-Getriebe-Einheit mündenden Antriebsspindel und mit einem Antriebsteil ausgerüstet sein, das durch ein Betätigungselement um seine Längsachse von der Antriebsspindel ausgeklinkt und dadurch unabhängig von der Motor-Getriebe-Einheit nach vorn oder zurück gezogen werden kann, wie dies in der DE-A 3428655 der Fall ist. Durch Wiedereinklinken des Antriebsteils kann dieses in einer beliebigen Stellung arretiert werden.

Infolge der festen Verbindung wird das positionsgebende Element mit dem Antriebsteil verschoben und gibt korreliert hierzu die Position des Antriebsteiles und damit des Spritzenkolbens wieder.

An einem Ausführungsbeispiel soll die Erfindung näher erläutert werden.

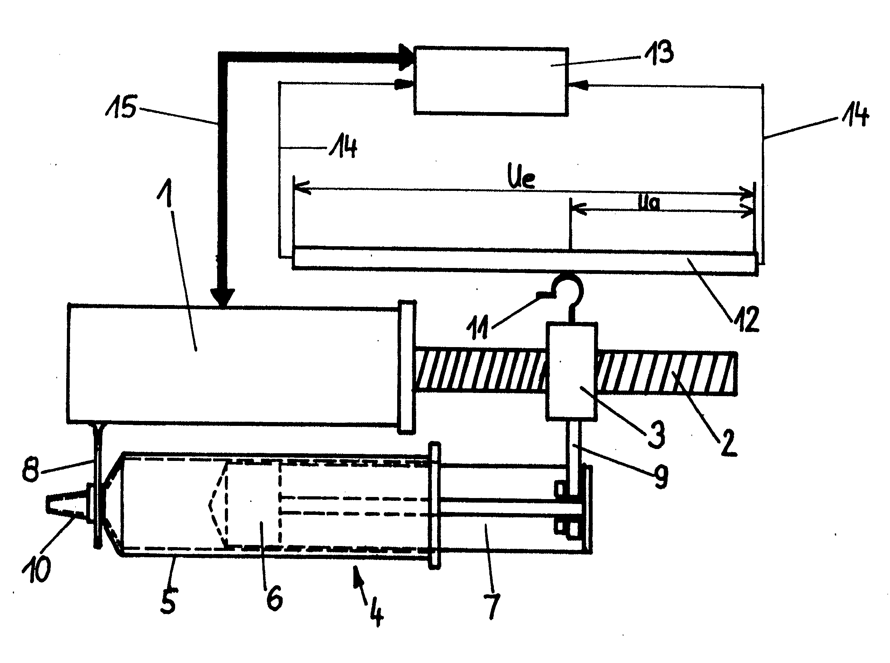

Dabei zeigt die beiliegende Zeichnung eine erfindungsgemäße für Infusionsspritzenpumpe, bei der der Spritzenkolben unabhängig von der Motor-Getriebe-Einheit in eine beliebige Ausgangsstellung bewegbar ist.

Die Infusionsspritzenpumpe weist eine Motor-Getriebe-Einheit 1 auf, die eine Gewindespindel 2 antreibt, die aus einer Stirnseite der Motor-Getriebe-Einheit 1 herausragt und auf der ein linear bewegbares Antriebsteil 3 angeordnet ist.

Die Infusionsspritze 4 besteht aus einem Spritzenzylinder 5 und einem Spritzenkolben 6 mit einer Kolbenstange 7. Das vordere Ende des Spritzenzylinders 5 ist in einen Halter 8 eingesetzt, der im Beispielsfalle an der Motor-Getriebe-Einheit 1 der Infusionsspritzenpumpe befestigt ist. Das rückwärtige Ende der Kolbenstange 7 ist in einen weiteren Halter 9 eingesetzt, der am linear bewegbaren Antriebsteil 3 angebracht ist. Wird das linear bewegbare Antriebsteil 3 in Richtung auf die Motor-Getriebe-Einheit 1 bewegt, wird der Spritzenkolben 6 im Spritzenzylinder 5 vorgeschoben und das Injektat wird durch den Spritzenauslaß 10 hindurch ausgeschoben. Der Spritzenauslaß ist über einen (nicht dargestellten) Schlauch mit dem Patienten verbunden.

Eine derartige Infusionsspritzenpumpe ist in der DE-A 3428655 beschrieben.

Am linear bewegbaren Antriebsteil 3 ist ein positionsgebendes Element 11 derart angeordnet, daß dieses mit einem Absolutwegaufnehmer 12 funktionell gekoppelt ist, insbesondere in Eingriff steht. Dieser Absolutwegaufnehmer 12 ist in einer besonderen erfindungsgemäßen Ausbildungsform als Linearpotentiometer ausgebildet, wie dies in der Zeichnung gezeigt ist. Der Absolutwegaufnehmer 12 kann auch durch ein bekanntes induktives, kapazitives oder magnetisches positionsgebendes Element gebildet werden.

Der Absolutwertaufnehmer 12 erstreckt sich, wie ebenfalls aus der Zeichnung ersichtlich, parallel zur Längsachse der Gewindespindel 2 bzw. zur Verschiebungsrichtung des Antriebsteiles 3.

Weiterhin ist der Absolutwegaufnehmer 12 mit einer Auswerteeinheit 13 über die Leitung 14 verbunden, die wiederum über eine Leitung 15 mit der Motor-Getriebe-Einheit 1 verbunden ist.

Ebenso sind die Enden des Linearpotentiometers über die Leitungen 14 mit der Auswerteeinheit 13 verbunden und bilden zusammen mit dem positionsgebenden Element 11, das als Schleifkontakt ausgebildet ist, einen Spannungsteiler.

Die Auswerteeinheit 13 gibt die Eingangsspannung $U_e$ des Absolutwegaufnehmers 12, nämlich des Linearpotentiometers, vor und speichert diese zugleich. Das positionsgebende Element 11 am linear bewegbaren Antriebsteil 3 befindet sich nun an einer bestimmten Position des Linearpotentiometers, das an dieser Stelle ein bestimmtes Ausgangsspannungssignal $U_a$ an die Auswerteeinheit 13 abgreift. Diese Ausgangsspannung $U_a$ ist proportional der Stellung des positionsgebendes Elementes 11 und damit der momentanen Stellung des Spritzenkolbens 6 im Spritzenzylinder 5 und gibt nach entsprechender Umwandlung seine Koordinate auf der Bewegungsbzw. Positionsachse unmittelbar an.

Infusionspumpen sind regelmäßig auf einen bestimmten Spritzentyp ausgelegt und sicherheitstechnisch auch nur für einen solchen Typ zugelassen. Insofern gibt die Ausgangsspannung $U_a$ für den zulässigen Spritzentyp die Absolutkoordinate des Spritzenkolbens an, sofern die Spritze ordnungsgemäß in die Aufnahmeeinrichtungen 8 und 9 eingelegt ist.

Andererseits kann aber auch ein anderer Spritzentyp nach einer entsprechenden Eichung eingesetzt werden.

In die Auswerteinheit werden vor bzw. während des Infusionsvorganges die notwendigen und üblichen infusionsrelevanten Parameter eingegeben. Insofern kann der am Linearpotentiometer abgegriffene Wert bereits die in der Spritze 5 verbliebene Restinfusionsmenge bzw. über die eingegebene Vorschubrate die verbleibende Restinfusionszeit angeben.

Die Schaltungen und Bauteile der Auswerteeinheit 13 entsprechen dabei dem bekannten Stand der Technik.

Im übrigen kann die Auswerteeinheit 13 — wie in der Zeichnung mit Leitung 15 bezeichnet — auch zur Steuerung der Motor-Getriebe-Einheit 1 herangezogen werden.

## Patentansprüche

1. Infusionsspritzenpumpe
   mit einer Motor-Getriebe-Einheit (1) zum Antrieb eines linear bewegbaren Antriebsteiles (3),
   mit einer Infusionsspritze (4), die einen Spritzenkolben (6) aufweist, der zu seiner Betätigung mit dem Antriebsteil (3) in Eingriff steht und

mit einer Positionserfassungseinrichtung zur Bestimmung der Stellung des Spritzenkolbens (6), die ein positionsgebendes Element (11) und einen ortsfesten Absolutwegaufnehmer (12) aufweist, dadurch gekennzeichnet,

daß das positionsgebende Element (11) mit dem linear bewegbaren Antriebsteil (3) starr verbunden ist, und

daß das positionsgebende Element (11) entlang seiner der Verschiebungsrichtung des Antriebsteils (3) entsprechenden Bewegungsbahn mit dem Absolutwegaufnehmer (12) elektrisch oder magnetisch gekoppelt ist.

2. Infusionsspritzenpumpe nach Anspruch 1, dadurch gekennzeichnet, daß der Absolutwegaufnehmer (12) als Linearpotentiometer ausgebildet ist.

3. Infusionsspritzenpumpe nach Anspruch 1, dadurch gekennzeichnet, daß der Absolutwegaufnehmer (12) ein induktives, kapazitives oder magnetisches positionsgebendes Element ist.

4. Infusionsspritzenpumpe nach den Ansprüchen 1 bis 3, dadurch gekennzeichnet, daß der Absolutwegaufnehmer (12) mit einer Auswerteeinheit (13) verbunden ist.

## Claims

1. Infusion syringe pump comprising

a motor-gear unit (1) for driving a linearly movable drive member (3),

an infusion syringe (4) having a syringe plunger (6) which for its actuation is in engagement with the drive member (3) and comprising

a position detecting means for determining the position of the syringe plunger (6), said means having a position-defining element (11) and a stationary absolute displacement pickup (12), characterized in

that the position-defining element (11) is rigidly connected to the linearly movable drive member (3), and

that the position-defining element (11) along its path of movement corresponding to the displacement direction of the drive member (3) is electrically or magnetically coupled to the absolute displacement pickup (12).

2. Infusion syringe pump according to claim 1, characterized in that the absolute displacement pickup (12) is constructed as linear potentiometer.

3. Infusion syringe pump according to claim 1, characterized in that the absolute displacement pickup (12) is constructed as inductive,capacitive or magnetic position-defining element.

4. Infusion syringe pump according to claims 1 to 3, characterized in that the absolute displacement pickup (12) is connected to an evaluation unit (13).

## Revendications

1. Pompe à seringue pour perfusion

comportant un ensemble moteur-engrenage (1) pour l'entraînement d'un élément d'entraînement (3) déplaçable linéairement,

comportant une seringue pour perfusion (4) qui comprend un piston de seringue (6) qui est en prise avec l'élément d'entraînement (3) en vue de son actionnement et

comportant un dispositif de détection de position pour déterminer la position du piston de seringue (6) qui comporte un élément (11) transmettant la position et un détecteur de parcours absolu (12) fixe, **caractérisée en ce que**

l'élément (11) transmettant la position est relié de manière rigide à l'élément d'entraînement (3) mobile linéairement et

en ce que l'élément (11) transmettant la position est couplé électriquement ou magnétiquement au détecteur de parcours absolu (12), le long de sa trajectoire de déplacement, correspondant à la direction de déplacement de l'élément d'entraînement (3).

2. Pompe de seringue pour perfusion selon la revendication 1, caractérisée en ce que le détecteur de parcours absolu (12) est un potentiomètre linéaire.

3. Pompe de seringue pour perfusion selon la revendication 1, caractérisée en ce que le détecteur de parcours absolu (12) est un élément inductif, capacitif ou magnétique transmettant la position.

4. Pompe de seringue pour perfusion selon les revendications 1 à 3, caractérisée en ce que le détecteur de parcours absolu (12) est relié à une unité d'exploitation (13).